# EUROPÄISCHE PATENTANMELDUNG

(11) **EP 3 375 429 A1**
(43) Veröffentlichungstag der Anmeldung: **19.09.2018**
(21) Anmeldenummer: 17161722.8
(22) Anmeldetag: 17.03.2017
(51) Int. Cl.: A61K 6/00, A61K 6/083

(54) **MONOCHROMATISCHER DENTALER FORMKÖRPER UND ROHLING ZUR HERSTELLUNG VON DENTALRESTAURATIONEN**

(71) Anmelder: Ivoclar Vivadent AG, 9494 Schaan (LI)
(72) Erfinder: Hagenbuch, Konrad, 9469 Haag (CH); Kerschbaumer, Harald, 6833 Klaus (AT); Gurschler, Konrad, 39020 Schnals (IT); Häfele, Clemens Andreas, 6833 Weiler (AT); Baaske, Thomas, 8885 Mels (CH)
(74) Vertreter: Uexküll & Stolberg

(57) **Zusammenfassung**

Einfarbiger dentaler Formkörper, der eine Opazität von 70 bis 78 % aufweist, und Verfahren zu seiner Herstellung.

## Beschreibung

Die vorliegende Erfindung betrifft einfarbige Formkörper, Rohlinge und dentale Formteile, Verfahren zu deren Herstellung und deren Verwendung zur Herstellung von dentalen Restaurationsformteilen. Die Formkörper bestehen vorzugsweise aus einem gefüllten oder ungefüllten Kunststoffmaterial und zeichnen sich durch eine vorgegebene Opazität aus.

Der natürliche Zahn weist einen Farbgradienten, Transluzenzunterschiede und unterschiedliche Farbhelligkeiten innerhalb des Zahnes auf. Um das Aussehen des natürlichen Zahns möglichst realistisch widerzugeben, sind Dentalrestauration daher meist ebenfalls mehrfarbig und haben einen zonaren Aufbau mit unterschiedlichen Bereichen, die dem natürlichen Zahn entsprechende Farben aufweisen.

Besonders hohe Anforderungen werden an Prothesen für den Frontzahnbereich gestellt. Hier werden standartmäßig künstliche Zähne mit mindestens zwei und meist drei bis vier Schichten eingesetzt, um eine natürlich wirkende Ästhetik zu erzielen. Diese Schichten setzen sich aus einer transparenten Schneide- einem opaken Dentin- und einem nochmals opakeren Hals-Material zusammen, wobei primär die Dentinschicht für die Farbwirkung verantwortlich ist.

Speziell bei künstlichen vorfabrizierten Prothesenzähnen muss somit ein hoher Aufwand betrieben werden, um eine ästhetische und farblich akzeptable Wirkung bei unterschiedlichen Zahngrössen und Zahnformen zu erreichen. Im Backenzahnbereich spielt die Ästhetik zwar eine weniger wichtige Rolle als bei Frontzähnen, gewinnt jedoch zunehmend an Bedeutung.

Im Zuge der Digitalisierung und der Einführung von CAD/CAM Verfahren wird auch zur Herstellung abnehmbarer Prothesen zunehmend auf patientenspezifisch gefertigte Prothesenzähne zurückgegriffen. Wenn Zähne und insbesondere durch mehrere zusammenhängende Zähne gebildete Zahnsegmente aus mehrschichtigen Rohlingen gefräst werden, ist es schwierig ein ästhetisch zufriedenstellendes Aussehen über die gesamte Restauration zu erzielen, weil die Restauration in der Regel nicht so platziert werden kann, dass alle Bereiche der Restauration in der für sie optimalen Schicht des Rohlings liegen. Zudem kann der Rohling nicht optimal ausgenutzt werden, was mit einer relativ großen Abfallmenge verbunden ist. Die Herstellung von mehr als einer Restauration aus einem Rohling ist daher nicht praktikabel. Selbst bei der Verwendung mehrschichtiger Rohlinge ist es üblich, die Restaurationen nach dem Fräsen zu verblenden.

Die DE 198 23 530 A1 offenbart quader- und zylinderförmige Blöcke aus Dentalharzmaterialien, die sich zur Herstellung von dentalen Prothesen wie Kronen oder Inlays durch Fräsverarbeitung eignen sollen. Das Dentalharzmaterial enthält Acrylharzpolymer und 20 bis 70 Gew.-% anorganischen Füllstoff mit einer mittleren Teilchengröße von 0,01 bis 0,04 µm. Zylinderförmige Blöcke haben beispielsweise einen Durchmesser von 15 mm und eine Höhe von 25 mm. Zur farblichen Anpassung an die umgebenden Zähne könnend die Restaurationen mit verblendet werden.

Aus der WO 00/40206 sind Rohlinge zur Herstellung von Dentalprothesen bekannt, die ein Polymerharz und ein fein verteiltes Füllstoffmaterial mit einem maximalen Partikeldurchmesser von 50 µm enthalten. Die Rohlinge zeichnen sich dadurch aus, dass sie frei von Rissen und so hergestellt sind, dass sie einen Thermoschocktest bestehen. Das Aussehen der Prothesen kann durch das Aufbringen zusätzlicher Materialien angepasst werden.

Der Erfindung liegt die Aufgabe zugrunde, dentale Formkörper und Rohlinge zur Herstellung von Dentalrestaurationen zur Verfügung zu stellen, die ein mit dem natürlichen Zahn übereinstimmendes Aussehen aufweisen und die einfach herzustellen und zu verarbeiten sind. Der Erfindung liegt insbesondere die Aufgabe zugrunde, hohen ästhetischen Ansprüchen genügende künstliche Zähne und Zahnsegmente und Rohlinge zur Verfügung zu stellen, die sich zur Herstellung von künstlichen Zähnen und Zahnsegmenten durch Fräsbearbeitung eignen.

Erfindungsgemäß wird diese Aufgabe durch einfarbige dentale Formkörper gelöst, die eine Opazität von 70 bis 78 % aufweisen. Die Formkörper haben vorzugweise die Form einer dentalen Restauration oder die Form eines Rohlings zur Herstellung einer dentalen Restauration. Die dentale Restauration ist vorzugsweise ein künstlicher Zahn oder ein Zahnsegment. Unter einem Zahnsegment wird ein Formkörper verstanden, der durch zwei oder mehr, vorzugsweise 2 bis 14 und insbesondere 2 bis 7 miteinander verbundene künstliche Zähne gebildet wird. Der Rohling eignet sich besonders zur Weiterverarbeitung durch spanabhebende Verfahren, wie die Fräsverarbeitung oder CAD/CAM-Verfahren.

Es wurde überraschend gefunden, dass ein einfarbiges Material in einem eng eingegrenzten Opazitätsbereich, die Herstellung von ästhetisch anspruchsvollen, natürlich wirkenden Dentalrestaurationen, ermöglicht. Diese Wirkung ist auf eine Art Chamäleoneffekt zurückzuführen, der auf einem Zusammenspiel von Opazität und äußerer Form der Formköper beruht. Bei zahnförmigen Formkörpern führen die Aussenkontur des Zahns und die damit verbundene Variation der Materialstärke innerhalb des gefundenen Opazitätsbereichs zu einem natürlich wirkenden Aussehen. Dort wo der Zahn dicker und damit die Schichtdicke größer ist, ist der Farbeffekt ausgeprägter und wirkt das Material opaker. Umgekehrt ist der Farbeffekt dort, wo der Zahn und damit die Schichten dünner sind, weniger ausgeprägt und das Material wirkt transparenter und heller. Insgesamt wird so durch eine einfache Maßnahme ein sehr natürlich wirkender Farb- und Transluzenzverlauf erzielt.

Erfindungsgemäß bevorzugt sind Formkörper mit einer Opazität von 72 bis 76 %, besonders bevorzugt 73 bis 75 % und ganz besonders bevorzug ca. 74%. Die Opazität O ist das Verhältnis von einfallendem Lichtstrom I₀ zu durchgehendem Lichtstrom I: O = I₀/I. Der Kehrwert der Opazität ist die Transmission. Erfindungsgemäß wird die Opazität an Prüfkörpern mit einem Durchmesser von 20 mm und einer Höhe von 2 ± 0,02 mm gemessen. Die Messung kann mit handelsüblichen Messgeräten erfolgen.

Grundsätzlich tritt der Effekt bei allen Materialien auf, die nach dem Einfärben auf einen zahnfarbenen Farbton eine Opazität innerhalb des erfindungsgemäßen Bereichs aufweisen. Die Formkörper können somit beispielsweise aus Dentalkeramik oder Kunststoff bestehen.

Erfindungsgemäß bevorzugt sind Formkörper aus Kunststoffen, die durch Polymerisation von organischen Monomeren erhalten werden. Die Kunststoffe können gefüllt oder ungefüllt sein. Unter gefüllten Kunststoffen werden Kunststoffe verstanden, die neben der polymerisierten Harzmatrix mindestens einen darin homogen verteilten Füllstoff enthalten. Die Formkörper werden dadurch erhalten, dass ein oder mehrere Füllstoffe in einem polymerisierbaren Harz homogen dispergiert werden. Die Mischung wird anschließend in die gewünschte Form gebracht und durch Polymerisation gehärtet. Die Härtung erfolgt vorzugsweise durch das Einbringen von Energie in Form von Temperatur, Druck oder elektromagnetischer Strahlung, die Verwendung kalthärtender Initiatorsysteme ist aber grundsätzlich ebenfalls möglich. Bevorzugt erfolgt die Härtung thermisch.

Als polymerisierbare Harze sind kationisch und insbesondere radikalisch polymerisierbare Monomere bevorzugt. Besonders bevorzugt werden als Harz ein oder mehrere monofunktionelle Monomere oder eine Mischung aus monofunktionellen und polyfunktionellen Monomeren eingesetzt. Unter monofunktionellen Monomeren werden Verbindungen mit einer, unter polyfunktionellen Monomeren Verbindungen mit zwei oder mehr, vorzugsweise 2 bis 4 und insbesondere 2 kationisch bzw. radikalisch polymerisierbaren Gruppen verstanden.

Als monofunktionelle radikalisch polymerisierbare Monomere lassen sich z.B. N-monosubstituierte Acrylamide, wie z.B. N-Ethylacrylamid einsetzen. Erfindungsgemäß bevorzugte monofunktionelle radikalisch polymerisierbare Monomere sind Mono(meth)acrylate. Besonders bevorzugte monofunktionelle (Meth)acrylate sind Methyl-, Ethyl-, 2-Hydroxyethyl-, Butyl-, Isobutyl-, Acetoxyacetylethyl- (AAE-), Benzyl-, Tetrahydrofurfuryl- Isobornyl- und p-Cumyl-phenoxyethylenglycol-(meth)acrylat (CMP-1E) und Mischungen davon, ganz besonders bevorzugt sind Isobutyl-, Tetrahydrofurfuryl- oder Isobornyl-(meth)acrylat, CMP-1E und Mischungen davon. Am meisten bevorzugt ist Methylmethacrylat (MMA).

Als polyfunktionelle radikalisch polymerisierbare Monomere lassen sich z.B. N- disubstitiuierte Acrylamide, wie z.B. N,N-Dimethylacrylamid, und Bisacrylamide, wie z.B. N,N'-Diethyl-1,3-bis(acrylamido)-propan, 1,3-Bis(methacrylamido)-propan, 1,4-Bis(acrylamido)-butan oder 1,4-Bis(acryloyl)piperazin einsetzen. Erfindungsgemäß sind polyfunktionelle und insbesondere difunktionelle (Meth)acrylate bevorzugt, wie z.B. Di-, Tri- oder Tetraethylenglycoldi(meth)acrylat, Trimethylolpropantri-(meth)acrylat, Pentaerythrittetra(meth)acrylat, sowie Glycerindi- und -tri(meth)acrylat, 1,4-Butandioldi(meth)-acrylat, 1,12-Dodecandioldi(meth)acrylat und Mischungen davon.

Besonders bevorzugte Dimethacrylate sind Bisphenol-A-dimethacrylat, 2,2-Bis[4-(2-hydroxy-3-methacryloyloxypropyl)-phenyl]propan (Bis-GMA; ein Additionsprodukt aus Methacrylsäure und Bisphenol-A-diglycidylether), ethoxy- oder propoxyliertes Bisphenol-A-Dimethacrylat, wie z.B. das Bisphenol-A-Dimethacrylat 2-[4-(2-Methacryloyloxyethoxyethoxy)phenyl]-2-[4-(2-methacryloyloxyethoxy)phenyl]propan) (SR-348c, Firma Sartomer), 2-(((2-(N-Methylacrylamido)ethoxy)carbonyl)amino)-ethylmethacrylat und 2,2-Bis[4-(2-methacryloxypropoxy)phenyl]-propan, Bis-(3-methacryloyloxymethyl)tricyclo-[5.2.1.0^{2,6}]decan und Mischungen davon. Ganz besonders bevorzugte polyfunktionelle Monomere sind Urethandimethacrylate (UDMA, 1,6-Bis-[2-methacryloyloxy-ethoxycarbonylamino]-2,2,4-trimethylhexan;
TMX-UDMA (ein Additionsprodukt aus einer Mischung von HEMA und Hydroxypropylmethacrylat (HPMA) mit α,α,α',α'-Tetramethyl-m-xylylendiisocyanat (TMXDI)),1,10-Decandioldimethacrylat (D₃MA), (Mono)-Ethylenglycoldimethacrylat und Mischungen davon.

Eine erfindungsgemäß besonders vorteilhafte Monomermischung ist eine Mischung der Monomere MMA, UMDA (oder TMX-UDMA) und Ethylenglycoldimethacrylat, wobei die Monomere bevorzugt in einem Gewichtsverhältnis von 9 bis 12 Gewichtsteilen MMA, 0,5 bis 2 Gewichtsteilen UDMA (oder TMX-UDMA) und 1 Gewichtsteil Ethylenglycoldimethacrylat eingesetzt werden.

Im Fall ungefüllter Materialien sind Kunststoffe auf der Basis von oder aus Polymethylmethacrylat (PMMA) bevorzugt.

Erfindungsgemäß sind jedoch Kunststoffe bevorzugt, die mindestens einen Füllstoff enthalten. Der oder die Füllstoffe können anorganischer, organisch-anorganischer oder vorzugsweise organischer Natur sein, wobei partikuläre Füllstoffe bevorzugt sind.

Bevorzugte anorganische partikuläre Füllstoffe sind Pulver röntgenopaker Gläser mit einer durchschnittlichen Teilchengröße von 0,01 bis 15 µm, vorzugsweise 0,10 bis 5,0 µm; röntgenopake Füllstoffe, wie Ytterbiumtrifluorid, mit einer durchschnittlichen Teilchengröße von 0,050 bis 2,0 µm, vorzugsweise 0,10 bis 1,0 µm; SiO₂, ZrO₂, ZnO sowie Mischoxide aus SiO₂, ZrO₂, ZnO und/oder TiO₂ mit einer durchschnittlichen Teilchengröße von 5 bis 500 nm, vorzugsweise 20 bis 200 nm; nanopartikuläre Füllstoffe, wie Tantal(V)-oxid, Bariumsulfat oder Mischoxide von SiO₂ mit Ytterbium(III)-oxid oder Tantal(V)-oxid mit einer durchschnittlichen Teilchengröße von 5 bis 500 nm, vorzugsweise 20 bis 200 nm. ZrO₂ und BaSO₄ eignen sich besonders gut zur Einstellung der Opazität.

Als organische Füllstoffe sind Polymerpartikel bevorzugt, insbesondere Partikel von vernetzten organischen Polymeren. Polymerpartikel können durch Härten eines oder mehrerer Monomere und anschließendes Mahlen der gehärteten Masse oder durch Suspensionspolymerisation erhalten werden. Die Härtung kann durch Licht oder thermisch erfolgen. Als Monomere sind radikalisch polymerisierbare Monomere und insbesondere die oben aufgeführten Monomere bevorzugt. Ganz besonders bevorzugt sind Dimethacrylate, speziell Bis-GMA, UDMA, TMX-UDMA, DCP, D₃MA und Mischungen davon.

Polymerpartikel, die durch Mahlen hergestellt werden, zeigen unter dem Mikroskop ein splitterförmiges Aussehen und werden daher auch als Splitterpolymerisate oder Splitterpolymere bezeichnet. Die durch Suspensionspolymerisation erhaltenen Perlpolymerisate zeichnen sich demgegenüber durch kugelförmige Partikel aus. Als organische Füllstoffe sind Splitterpolymerisate bevorzugt.

Unter organisch-anorganischen Füllstoffen werden Polymerpartikel verstanden, die ihrerseits mit anorganischen Füllstoffen gefüllt sind. Derartige Füller werden auch als Kompositfüller bezeichnet. Organisch-anorganische Füllstoffe werden vorzugsweise durch Härtung von Massen auf der Basis von radikalisch polymerisierbaren Monomeren und anorganischen Füllstoffen hergestellt. Die Härtung kann lichtinduziert oder vorzugsweise thermisch erfolgen. Dabei kommen als radikalisch polymerisierbare Monomere vorzugsweise Dimethacrylate und besonders bevorzugt Bis-GMA, UDMA, TMX-UDMA, DCP und/oder D₃MA zum Einsatz. Als Füllstoffe dienen vorzugsweise pyrogene oder Fällungskieselsäuren, röntgenopake Glasfüllstoffe, Ytterbiumtrifluorid und Mischungen dieser Komponenten. Der Brechungsindex der polymerisierten Monomermischung wird vorzugsweise so eingestellt, dass er dem Brechungsindex des verwendeten Hauptfüllstoffs weitgehend entspricht. Auf diese Weise wird erreicht, dass der Kompositfüller eine hohe Transparenz aufweist. Die Polymerisate werden gemahlen und als Pulver eingesetzt.

Die organischen oder organisch-anorganischen Füllstoffe weisen vorzugsweise eine mittlere Teilchengrößen von 2 bis 100 µm, besonders bevorzugt 2 bis 60 µm oder 5 bis 60 µm, ganz besonders bevorzugt von 10 bis 50 µm am meisten bevorzugt von 10 bis 30 µm und insbesondere ca. 20 µm auf. Bei allen Partikelgrößen von Splitter- oder Perlpolymerisaten handelt es sich um gemittelte d50 Werte, die z.B. auf einem Partikelgrößenmessgerät des Typs Laser Particle Size Analyzer Horiba LA-960 (HORIBA Ltd. Kyoto/Japan) gemessen werden können. Die Messung erfolgt mittels Laserbeugungsspektroskopie. Die zu analysierenden Proben werden in einem geeigneten Hilfsmittel wie z.B. einer 20-%-igen wässrigen Lösung von Natriumpolyphosphat (anorganische Proben) oder einer wässrigen 0,5 Gew.-%igen Tween-Lösung (Polyoxyethylensorbitanmonolaurat-Lösung) dispergiert. Die Berechnungen der Resultate erfolgen nach den Regeln der Mie-Theorie.

Um ein möglichst naturgetreues Aussehen von Dentalrestaurationen zu erzielen, enthalten die erfindungsgemäßen Kunststoffe vorzugsweise mindestens einen Kompositfüller und besonders bevorzugt mindestens einen organischen Füllstoff, der eine höhere Transparenz, d.h. eine geringere Opazität, als das Matrixmaterial aufweist. Besonders bevorzugt sind die oben beschriebenen vernetzten Polymerpartikel. Diese Füllstoffe weisen eine relativ große Teilchengröße auf und sind im Stande, Licht tief in das Innere des Materials zu leiten, so dass ein gewisser Tiefeneffekt entsteht, der der Restauration eine natürliche Optik verleiht.

Die gefüllten oder ungefüllten Kunststoffe enthalten vorzugsweise mindestens ein Farbmittel, wobei als Farbmittel Pigmente bevorzugt sind. Besonders bevorzugt sind Materialen, die ein oder mehrere Pigmente enthalten, die aus den folgenden Stoffen ausgewählt sind:

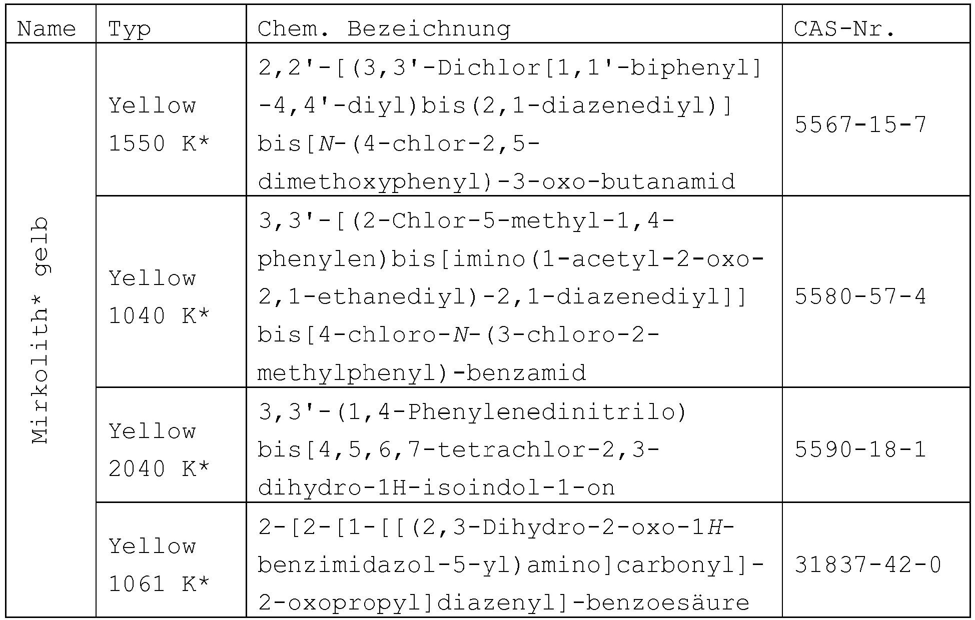

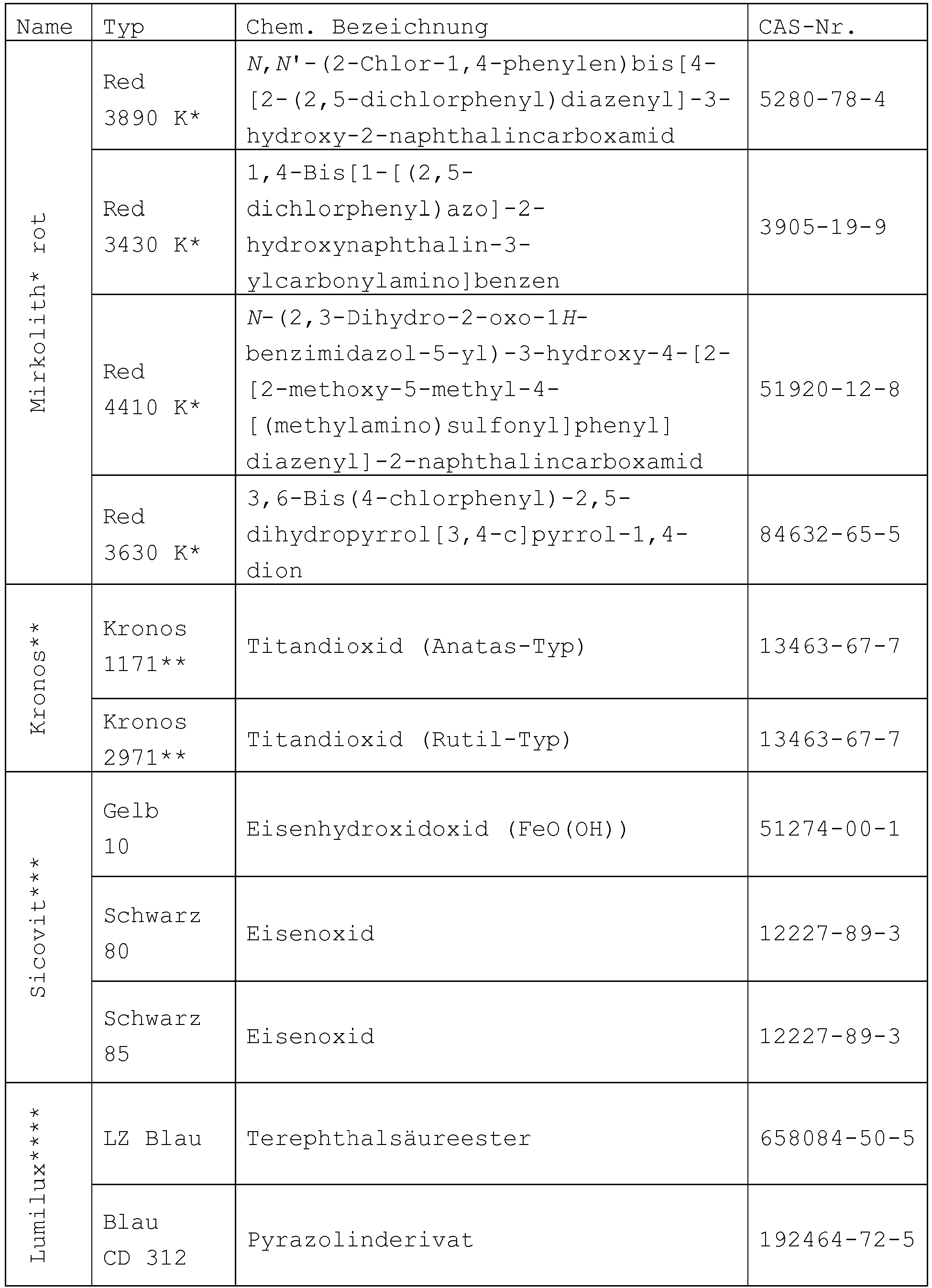

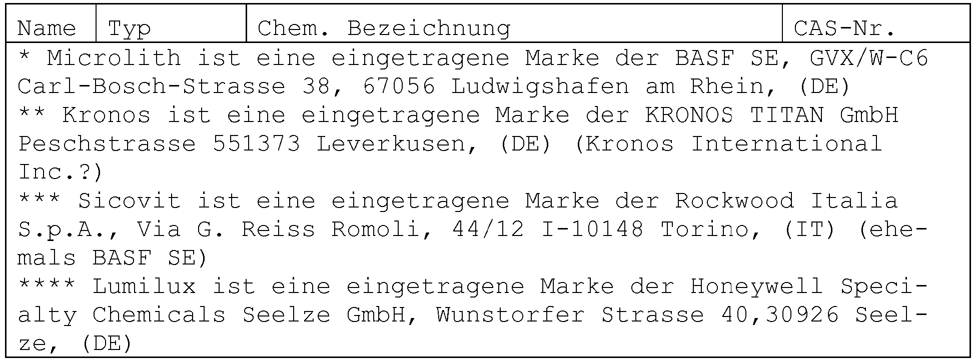

Besonders bevorzugte Pigmente sind Eisenoxid Schwarz, Eisenhydroxidoxid gelb, TiO₂ Weiss, Microlith rot, Microlith gelb und Mischungen davon.

Die Farbmittel werden vorzugsweise so abgestimmt, dass die Materialen nach dem Härten zahnfarben sind. Besonders bevorzugt weisen die Formkörper nach dem Härten eine der folgenden Farben auf:

| **Farbe** | **L*a*b* - Werte** | | |
|---|---|---|---|
| | **L** | **a** | **b** |
| **BL3** | 86.0 | 3.4 | 15.5 |
| **A1** | 84.0 | 5.0 | 22.3 |
| **A2** | 79.5 | 7.7 | 26.4 |
| **A3** | 78.5 | 8.3 | 29.0 |
| **A3,5** | 76.5 | 9.5 | 31.4 |
| **B1** | 82.5 | 3.8 | 21.4 |
| **B3** | 78.0 | 7.4 | 32.0 |
| **C2** | 75.5 | 6.3 | 25.4 |
| **D2** | 77.5 | 5.9 | 22.3 |

Die Farben werden gemäß dem L*a*b*-Farbmodell entsprechend DIN EN ISO 11664-4 bestimmt. Die Farbmessung kann mit handelsüblichen Messgeräten wie dem Minolta CM-3700d Farbmessgerät durchgeführt werden.

Die Farbbezeichnungen A1, A2, A3 usw. entsprechen dem Vita Farbsystem (Vita Farbschlüssel Lumin-VACUUM). Hierbei handelt es sich um eine auf dem Dentalgebiet übliche und weitverbreitete Farbskala. Diese Bezeichnungen erlauben dem Fachmann eine schnelle Zuordnung der Farben.

Die Kunststoffe enthalten weiterhin vorzugsweise ein Verdickungsmittel. Als Verdickungsmittel sind unvernetzte Polymerpartikel bevorzugt, insbesondere Perlpolymerisate, die vorteilhaft durch Suspensionspolymerisation hergestellt werden können. Als Monomere sind radikalisch polymerisierbare Monomere und insbesondere die oben aufgeführten Monomere bevorzugt. Ganz besonders bevorzugt sind Mono(meth)acrylate, insbesondere Methylmethacrylat (MMA). Die unvernetzten Polymerpartikel haben im Ausgangszustand vorzugsweise eine mittlere Partikelgröße von 10 bis 120 µm, besonders bevorzugt 10 bis 60 µm und ganz besonders bevorzugt ca. 50 µm. Die unvernetzten Polymerpartikel unterscheiden sich den als Füllstoff eingesetzten vernetzten Polymerpartikeln dadurch, dass sie in den als Matrixmaterial verwendeten Monomeren zumindest teilweise löslich sind und so ihre Partikelgröße andern können. Sie werden daher nicht als Füllstoffe angesehen.

Zur Herstellung von Formkörpern werden die genannten Komponenten und ggf. weitere Additive homogen miteinander gemischt, die Mischung wird in die gewünschte Form gebracht und dann durch Polymerisation gehärtet.

Als weitere Additive kommen insbesondere Initiatoren für die radikalische und/oder kationische Polymerisation, Stabilisatoren, wie z.B. Polymerisationsstabilisatoren, antibakterielle Wirkstoffe, fluoridionenabgebende Additive, optische Aufheller, Fluoreszenzmittel, UV-Absorber, Entschäumer, Thixotropiehilfsmittel, Stoffe zur Verbesserung der Bruchzähigkeit und/oder Effektmittel in Betracht.

Als Initiatoren für die beschriebenen Systeme kommen dem Fachmann bekannte thermische, wie auch strahlungsaktivierte Initiatoren in Frage. Gebräuchliche thermische Initiatoren sind Lauroylperoxid, tert.Butylperoxid, Cumolhydroperoxid, oder auch Azoisobutyronitril. Besonders bevorzugt ist Benzoylperoxid, welches auch, wie in Dentalkunststoffen gebräuchlich in den PMMA-Perlpolymeren verkapselt vorliegen kann. Kombinationen mit tertiären, meist aromatischen Aminen wie N,N-Dimethylp-toluidin oder N,N-Diethanol-p-toluidin (DEPT) als Beschleuniger können als bei Raumtemperatur startende Initatorsysteme herangezogen werden. Bekannte Photosensibilisatoren sind Diketone, wie z.B. 9,10-Phenanthrenchinon, 1-Phenyl-propan-1,2-dion, Diacetyl oder 4,4'-Dichlorbenzil oder deren Derivate, besonders bevorzugt ist Campherchinon (CC) und dessen Derivate, und Mischungen davon.

Vorzugsweise werden die angeführten Photoinitiatoren in Kombination mit Beschleunigern eingesetzt. Als Beschleuniger für photoaktive Systeme eignen sich besonders tertiäre Amine, wie z.B. tertiäre aromatische Amine, insbesondere N,N-Dialkylaniline, -p-toluidine oder -3,5-xylidine, p-(N,N-Dialkylaminophenylethanol, -benzoesäurederivate, -benzaldehyd, -phenylessigsäureester und -phenylpropionsäureester. Konkrete Beispiele hierfür sind N,N-Dimethylanilin, N,N-Dimethyl-p-toluidin, N,N,3,5-Tetramethylanilin, N,N-Dimethylamino-p-benzaldehyd, p-(Dimethylamino)-benzoesäureethylester oder p-(Dimethylamino)-benzonitril. Geeignet sind auch tertiäre aliphatische Amine, wie z.B. Tri-n-butylamin, Dimethylaminoethan-2-ol, Triethanolamin, Dimethylaminoethylmethacrylat, N,N-Dimethylbenzylamin, oder heterocyclische Amine, wie z.B. 1,2,2,6,6-Pentamethylpiperidin, und Aminosäure-Derivate, wie z.B. N-Phenylglycin. Besonders bevorzugte Photoinitiatoren sind Acyl- oder Bisacylgermanium-Verbindungen, insbesondere die in der EP 1 905 413 A1 offenbarten Monoacyltrialkyl- und Bisacyldialkylgermanium-Verbindungen, wie z.B. Benzoyltrimethylgermanium, Bisbenzoyldiethylgermanium oder Bis(4-methoxybenzoyl)diethylgermanium. Acyl- und Bisacylgermanium-Verbindungen haben den Vorteil, dass sie sich nach der Bestrahlung entfärben (Bleaching Effekt) und so bei längerem Gebrauch der mittels dieser hergestellten Formkörper, keine Farbveränderungen auftreten. Außerdem handelt es sich um monomolekulare Photoinitiatoren, d.h. sie benötigen keinen Beschleuniger, um ihre volle Aktivität zu erreichen. Weitere besonders bevorzugte Photoinitiatoren sind Acyl- oder Bisacylphosphinoxide, insbesondere die in EP 0 007 505, EP 0 073 413, EP 0 184 095 und EP 0 615 980 beschriebenen Verbindungen. Bevorzugte Beispiele sind die kommerziell zugänglichen Verbindungen 2,4,6-Trimethylbenzoyldiphenylphosphinoxid (Lucirin□ TPO, BASF) und Bis(2,4,6-trimethylbenzoyl)phenylphosphinoxid (Irgacure□ 819, Ciba). Acyl- und Bisacylphosphinoxide gehören ebenfalls zur Gruppe der monomolekularen Photoinitiatoren und zeichnen sich durch eine geringe Eigenabsorption aus.

Zur Einstellung der gewünschten Opazität geht man dabei vorzugsweise so vor, dass man Harz und ggf. Füllstoff mischt, durch die Zugabe des oder der Farbmittel einfärbt, dann die Opazität misst und anschließend einstellt.

Das Pigment Titandioxid, weist einen Brechungsindex n_{D}²⁰ von 2,52 auf, der deutlich über dem der meisten Matrixmaterialien liegt, und erlaubt so schon bei der Einfärbung eine weitgehende Einstellung der Opazität.

Sollte die Opazität nach der Einfärbung noch unterhalb der oben definierten Werte liegen, kann sie durch die Zugabe eines Füllstoffs erhöht werden, dessen Brechungsindex von dem des Matrixmaterials abweicht. Vorzugsweise erfolgt die Einstellung mit feinteiligen anorganischen Füllstoffen wie ZrO₂ und/oder BaSO₄ mit einer Teilchengröße von 5 bis 500 nm, vorzugsweise 20 bis 200 nm. Sollte die gemessene Opazität zu hoch sein, kann sie durch Zugabe von Matrixmaterial oder eines Füllers mit einem Matrix-ähnlichen Brechungsindex verringert werden.

Die Mischung kann auf unterschiedliche Weise zu Formkörpern geformt werden. Gemäß einer Ausführungsform wird die Mischung in eine geeignete Gießform gefüllt und dann gehärtet. Auf diese Weise können z.B. quader-, würfel- oder zylinderförmige Rohlinge oder Rohlinge mit einer anderen dreidimensionalen Form wie Prothesenzähne und Zahnsegmente hergestellt werden. Bei dieser Ausführungsform werden die Mischungen vorzugsweise thermisch gehärtet.

Gemäß einer alternativen Ausführungsform wird die Mischung durch generative Verfahren zu Formkörpern geformt und dabei vorzugsweise schichtweise gehärtet. Ein bevorzugtes generatives Verfahren ist die Stereolithographie. Bei generativen Verfahren erfolgt die Härtung vorzugsweise durch Licht.

Mischungen, die sich besonders für Gießverfahren eignen, weisen vorzugsweise die folgende Zusammensetzung auf:
(1) 1 bis 20 Gew.-%, vorzugsweise 2 bis 15 Gew.-% und ganz besonders bevorzugt 3 bis 12 Gew.-% polyfunktionelle(s) radikalisch polymerisierbare(s) Monomer(e),
(2) 10 bis 60 Gew.-%, vorzugsweise 5 bis 50 Gew.-% und ganz besonders bevorzugt 10 bis 45 Gew.-% monofunktionelle(s) radikalisch polymerisierbare(s) Monomer(e),
(3) 0,01 bis 5,0 Gew.-%, vorzugsweise 0,1 bis 3,0 Gew.-% und ganz besonders bevorzugt 0,1 bis 2,0 Gew.-% Initiator für die radikalische Polymerisation,
(4) 5 bis 90 Gew.-%, vorzugsweise 10 bis 85 Gew.-% und ganz besonders bevorzugt 20 bis 80 Gew.-% Füllstoff(e), und ggf.
(5) 0,1 bis 5,0 Gew.-%, vorzugsweise 0,1 bis 2,0 Gew.-% und ganz besonders bevorzugt 0,2 bis 1,5 Gew.-% weitere Additiv(e), jeweils bezogen auf die Gesamtmasse der Mischung.

Bevorzugt sind Mischungen, die die folgende Zusammensetzung aufweisen:
(1) 1 bis 20 Gew.-%, vorzugsweise 2 bis 15 Gew.-% und ganz besonders bevorzugt 3 bis 12 Gew.-% polyfunktionelle(s) radikalisch polymerisierbare(s) Monomer(e),
(2) 10 bis 60 Gew.-%, vorzugsweise 5 bis 50 Gew.-% und ganz besonders bevorzugt 10 bis 45 Gew.-% monofunktionelle(s) radikalisch polymerisierbare(s) Monomer(e),
(3) 0,01 bis 5,0 Gew.-%, vorzugsweise 0,1 bis 3,0 Gew.-% und ganz besonders bevorzugt 0,1 bis 2,0 Gew.-% Initiator für die radikalische Polymerisation,
(4) 20 bis 90 Gew.-%, vorzugsweise 35 bis 80 Gew.-% und ganz besonders bevorzugt 45 bis 70 Gew.-% Verdickungsmittel, vorzugsweise unvernetzte Polymerpartikel, und ggf.
(5) 0,1 bis 5,0 Gew.-%, vorzugsweise 0,1 bis 2,0 Gew.-% und ganz besonders bevorzugt 0,2 bis 1,5 Gew.-% weitere Additiv(e), jeweils bezogen auf die Gesamtmasse der Mischung.

Besonders bevorzugt sind Mischungen, die die folgende Zusammensetzung aufweisen:
(1) 1 bis 20 Gew.-%, vorzugsweise 2 bis 10 Gew.-% und ganz besonders bevorzugt 3 bis 8 Gew.-% polyfunktionelle(s) radikalisch polymerisierbare(s) Monomer(e),
(2) 10 bis 60 Gew.-%, vorzugsweise 5 bis 50 Gew.-% und ganz besonders bevorzugt 10 bis 45 Gew.-% monofunktionelle(s) radikalisch polymerisierbare(s) Monomer(e),
(3) 0,01 bis 5,0 Gew.-%, vorzugsweise 0,1 bis 3,0 Gew.-% und ganz besonders bevorzugt 0,1 bis 2,0 Gew.-% Initiator für die radikalische Polymerisation,
(4) 20 bis 70 Gew.-%, vorzugsweise 30 bis 60 Gew.-% und ganz besonders bevorzugt 35 bis 55 Gew.-% Verdickungsmittel, vorzugsweise unvernetzte Polymerpartikel,
(5) 5 bis 50 Gew.-%, vorzugsweise 10 bis 40 Gew.-% und ganz besonders bevorzugt 15 bis 35 Gew.-% organischen Füllstoff, vorzugsweise vernetzte Polymerpartikel und ggf.
(6) 0,1 bis 5,0 Gew.-%, vorzugsweise 0,1 bis 2,0 Gew.-% und ganz besonders bevorzugt 0,2 bis 1,5 Gew.-% weitere Additiv(e), jeweils bezogen auf die Gesamtmasse der Mischung.

Bevorzugte Mischungen, die sich besonders für generative Verfahren eignen, weisen vorzugsweise die folgende Zusammensetzung auf:
(1) 15 bis 70 Gew.-%, vorzugsweise 20 bis 60 Gew.-% und ganz besonders bevorzugt 25 bis 50 Gew.-% mono- und/oder polyfunktionelle(s) radikalisch polymerisierbare(s) Monomer(e),
(2) 0,01 bis 5,0 Gew.-%, vorzugsweise 0,1 bis 3,0 Gew.-% und ganz besonders bevorzugt 0,1 bis 2,0 Gew.-% Photoinitiator für die radikalische, kationische oder kombinierte Polymerisation,
(3) 5 bis 90 Gew.-%, vorzugsweise 10 bis 85 Gew.-% und ganz besonders bevorzugt 30 40 bis 80 Gew.-% Füllstoff(e), und ggf.
(4) 0,1 bis 5,0 Gew.-%, vorzugsweise 0,1 bis 2,0 Gew.-% und ganz besonders bevorzugt 0,2 bis 1,5 Gew.-% weitere Additiv(e), jeweils bezogen auf die Gesamtmasse der Mischung.

Die Verwendung der ungehärteten Mischungen zur Herstellung von Formkörpern durch aufbauende bzw. generative Verfahren ist ebenfalls Gegenstand der Erfindung.

Gegenstand der Erfindung ist auch ein Verfahren zur Herstellung von Formkörpern, bei dem man:
(a) eine polymerisierbare Mischung durch Mischen von polymerisierbarer Matrix, ggf. Füllstoff und ggf. Additiven bereitstellt,
(b) man die Mischung durch die Zugabe von Farbmitteln einfärbt,
(c) man ggf. die Opazität der Mischung ggf. durch die Zugabe feinkörnigen Füllstoffs, vorzugsweise von ZrO₂ und/oder BaSO₄ einstellt,
(d) man die Mischung zu einem Formkörper formt und
(e) durch radikalische und/oder kationische Polymerisation härtet.

Vorzugsweise unterwirft man den Formkörper nach der Härtung einer Nachvergütung (Schritt f), z.B. durch Tempern bei erhöhten Temperaturen, idealerweise im Temperaturbereich von 20 bis 90 °C und/oder Bestrahlung mit Licht, idealerweise mit Wellenlängen von 300 bis 500 nm. Durch die Nachvergütung wird das Ausmaß der Polymerisation erhöht und der Gehalt an Restmonomer verringert.

Gemäß einer bevorzugten Ausführungsform der Erfindung formt man den Formkörper in Schritt (d), indem man die Mischung durch Spritzgießen in eine Gießform, beispielsweise eine Stahlform, einbringt und dann in Schritt (e) härtet. Die Härtung erfolgt vorzugsweise thermisch, z.B. in einer Hydraulikpresse für 20 Minuten bei einer Temperatur von 140°C und einem Druck von 30 bar

Gemäß einer weiteren bevorzugten Ausführungsform der Erfindung wird die Mischung in Schritt (d) durch ein generatives Verfahren, vorzugsweise durch Stereolithographie zu einem Formkörper geformt. Der Formkörper kann nach der Fertigstellung insgesamt oder während der Herstellung schichtweise durch radikalische und/oder kationische Polymerisation gehärtet werden. Die schichtweise Härtung erfolgt vorzugsweise photochemisch. Generative Verfahren eignen sich besonders zur Herstellung von Formkörpern, die die Form von Zähnen oder Zahnsegmenten haben.

Generative Fertigungsverfahren werden auch als aufbauende, auftragende oder additive Verfahren bezeichnet. Üblich sind auch die Begriffe "Additive Manufacturing" oder "3D-Druck".

Die Formkörper haben vorzugsweise die Form von Zähnen, Zahnsegmenten, Blöcken oder Scheiben. Formkörper zur Verwendung als Rohlingen haben vorzugsweise die Form von zylinderförmigen Blöcken mit einem Durchmesser von 80 bis 110 mm, vorzugsweise 90 bis 100 mm, insbesondere ca. 100 mm und einer Dicke von 10 bis 30 mm. Diese zylinderförmigen Blöcke eignen sich besonders als Rohlinge zur Anfertigung von Dentalrestaurationen wie Zähnen oder Zahnsegmenten durch spanabhebende Verfahren. Hierzu wird der Rohling beispielsweise im Dentallabor oder einer zahnärztlichen Klinik zu einem dentalen Formteil geformt. Dies kann vorzugsweise durch Fräsen oder Schleifen mittels einer CAD/CAM-Anlage erfolgen. Die aus diesen Scheiben gefrästen Zähne und Zahnsegmente, zeichnen sich durch eine hervorragende Ästhetik aus und können ohne weitere Bearbeitung, d.h. insbesondere ohne Verblendung, zur Herstellung von Prothesen eingesetzt werden.

Die Erfindung betrifft auch die Verwendung der erfindungsgemäßen Rohlinge oder Formkörper zur Herstellung von dentalen Restaurationen. Die dentale Restauration ist dabei vorzugsweise ein Zahn oder ein Zahnsegment oder eine Teil- oder Vollprothese, die unter Verwendung dieser Zähne oder Zahnsegmente hergestellt wurde.

Die erfindungsgemäßen Materialen sind in mehrfacher Hinsicht vorteilhaft. Die erfindungsgemäßen Formkörper weisen keinen Schichtaufbau sondern eine homogene Zusammensetzung auf, was die Herstellung von Rohlingen und künstlichen Zähnen vereinfacht. Zudem werden Verbundprobleme zwischen den Schichten vermieden. Die Formkörper zeichnen sich durch eine hohe Materialhomogenität aus, so dass Spannungen im Material vorgebeugt wird. Im Fall von Rohlingen können die zu fräsenden Restaurationen ohne schichtbedingte Einschränkungen frei positioniert werden, was eine optimale Ausnutzung der Rohlinge ermöglicht, beispielsweise das Fräsen von mehreren Restaurationen aus einem Rohling, und unnötigen Abfall vermeidet. Es ist eine maximale Fräsfreiheit gegeben, was die Herstellung großer Rohlinge wirtschaftlich macht. Bei der Herstellung von Zahnsegmenten ist die Einfarbigkeit vorteilhaft, weil Verbinder zwischen den miteinander verbundenen Zähnen weniger auffallen.

Bei aufbauenden/additiven Fertigungsverfahren kann das gesamte Verfahren mit einem Material durchgeführt werden. Es ist kein Materialwechsel während des Bauprozesses erforderlich, und die Datenbereitstellung kann wesentlich vereinfacht werden, weil nur eine Umhüllende für das gesamte Bauteil benötigt wird.

Die erfindungsgemäß angestrebten Vorteile werden dann besonders deutlich, wenn der Formkörper eine Zahngeometrie, d.h. gleichzeitig Bereiche mit großer und geringer Schichtdicke aufweist. Die Herstellung von Formkörpern mit geringer Schichtdicke wie Kronen und Veneers (Schalen) ist möglich, erfordert aber, dass die Zahnstümpfe im Mund des Patienten mit einer farblich abgestimmten Opaquerschicht überzogen werden.

Die aus den erfindungsgemäßen Materialen gefertigten Zähne und Zahnsegmente eignen sich im Besonderen für die Herstellung von Frontzähnen für abnehmbare Total- und Teilprothesen. Die Zähne zeigen zudem eine hohe Übereinstimmung mit den auf dem Dentalgebiet standardmässig eingesetzten Farbschlüsseln und sind mit kommerziell erhältlichen Kunstzähnen kompatibel, was im Hinblick auf die Reparatur von vorhandenen Prothesen vorteilhaft ist.

Im Folgenden wird die Erfindung anhand von Figuren und Ausführungsbeispielen genauer beschrieben.

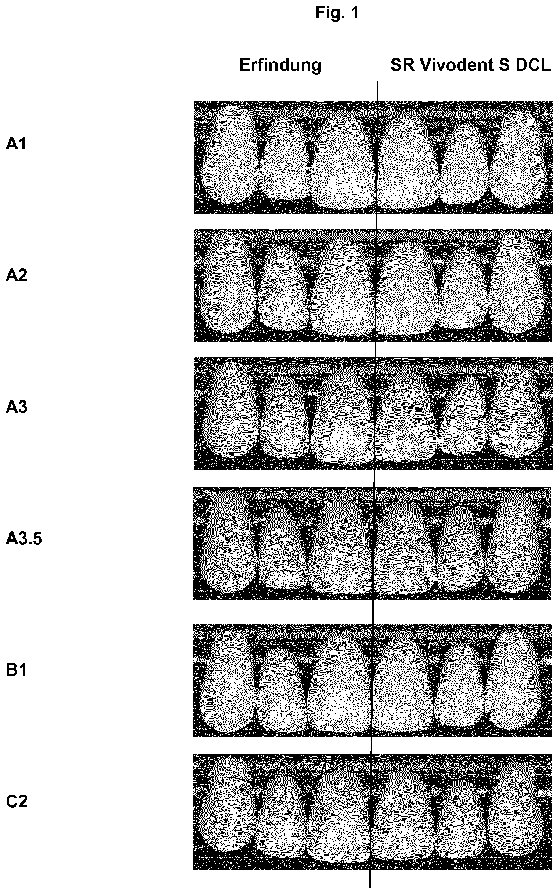

Figur 1 zeigt aus erfindungsgemäßen Rohlingen gefräste Zähne im Vergleich zu handelsüblichen Kunststoffzähnen der gleichen Farbe mit einem mehrschichtigen Aufbau.

### Ausführungsbeispiele

### Beispiel 1

### Herstellung einer polymerisierbaren Zusammensetzung zur Herstellung von Formkörpern

Die in Tabelle 1 angegebenen Komponenten wurden zu einer homogenen Masse miteinander gemischt. Um eine möglichst homogene Pigmentverteilung zu erreichen, wurden die Pigmente zunächst mit den unvernetzten Polymerpartikeln homogen vermischt und diese Mischung dann mit den übrigen Komponenten gemischt. Aus der entstandenen Masse wurden Prüfkörper mit einem Durchmesser von 20 mm und einer Höhe von 2 ± 0,02 mm polymerisiert. Die Polymerisation erfolgte in einem Druckpolymerisationsgerät (Ivomat IP3, Fa. Ivoclar Vivadent AG) bei 120°C unter Wasser bei einem Druck von 6 bar für 15 min. Die erhaltenen Prüfkörper wurden vor der Farb- und Opazitätsmessung mit einer Nassschleifmaschine mit Schleifpapier (Körnung P4000) nass poliert. Die Opazitäts- und die Farbmessung erfolgten mit einem Minolta CM-3700d Farbmessgerät. Die Farbe wurde entsprechend dem L*a*b*-Farbmodell gemäß DIN EN ISO 11664-4 gemessen. Die Ergebnisse werden in Tabelle 3 gezeigt.

**Tabelle 1: Zusammensetzung zur Herstellung von Formkörpern**

| **Komponente** | | **Bsp. 1** |
|---|---|---|
| Monomer | Methylmethacrylat (MMA) | 80 g |
| | CAS 80-62-6 | |
| | Urethandimethacrylat(UDMA) | 12,5 g |
| | CAS 72869-86-4 | |
| | Ethylenglykoldimethacrylat | 7,5 g |
| | CAS 97-90-5 | |
| Verdickungsmittel | PMMA-Pulver (mittlere Korngrösse 40 bis 60 µm)* | 119 g |
| | CAS (PMMA) 9011-14-7 | |
| Füllstoff | Polymer-Pulver (UDMA polymerisiert und gemahlen auf mittlere Korngrösse von 20 µm) | 80 g |
| Initiator | Benzoylperoxid | 1 g |
| | CAS 94-36-0 | |

| | | |
|---|---|---|
| * Mischung von unvernetzten Perlpolymerpartikeln und Pigmenten gemäß Tabelle 2 | | |

**Tabelle 2: Zusammensetzung der Mischung von PMMA-Pulver und Pigmenten**

| **Komponenten** | **Gewichts-%** |
|---|---|
| PMMA Perlpolymer | 99.8606 |
| TiO₂-Weisspigment | 0.1200 |
| Mikrolith-Gelb-Typen | 0.0089 |
| Sicovit-Gelb | 0.0032 |
| Sicovit-Schwarz | 0.0022 |
| Microlith-Rot | 0.0029 |
| Lumilux Fluoreszenzmittel | 0.0022 |
| Summe | 100.0000 |

**Tabelle 3: Farbe und Opazität von Bsp. 1**

| **Farbe *⁾** | **L*a*b*-Werte gemäß L*a*b*-Farbsystem** | | | **Opazität** |
|---|---|---|---|---|
| A3 | L | A | B | |
| | Helligkeit | rot-grün | gelb-blau | |
| Bsp. 1 | 77.51 | 9.03 | 29.71 | 73.95 |

| | | | | |
|---|---|---|---|---|
| *) Farbbezeichnung gemäß Vita Farbschlüssel Lumin-VACCUM | | | | |

In Beispiel 1 wurde durch die Kombination von PMMA-Pulver (Verdickungsmittel) und organischem Füllstoff (vernetztem Polymer; Splitterpolymer) die gewünschte Opazität erzielt. Die Formkörper zeichnen sich durch eine natürliche Transluzenz, Lichtstreuung und Farbwirkung aus.

### Beispiel 2

### Herstellung von zahnförmiger Formkörpern

Die Herstellung zahnförmiger Formkörper wurden zunächst zylinderförmige Rohlinge mit passender Dimension hergestellt und daraus anschließend Prothesenzähne gefräst. Die Rohlinge wurden aus Materialien basierend auf der Rezeptur aus Beispiel 1 hergestellt, die den Anforderungen entsprechend eingefärbt wurden. Nach Einstellung der Grundfarbe wurde die Opazität anhand von Prüfkörpern wie in Beispiel 1 beschrieben gemessen. Die verbliebene Pulvermischung dann durch die Zugabe von weiterem TiO₂ auf die in Tabelle 4 genannten Opazitätswerte eingestellt. Die Polymerisation des Materials zu Scheiben erfolgte nach dem Anteigen der gefärbten Pulvermischungen mit Monomer (Mischungsverhältnis siehe Tabelle 1) jeweils in einer Stahlform mittels einer hydraulischen Heisspresse bei 140°C und einem Druck von 30 bar für 20 min. Aus den so hergestellten Scheiben wurden mit einer Dentalfräse (Zenotec Select Ion, Wieland Dental) Zähne mit der Aussengeometrie bestehender konfektionierter Prothesenzähne gefräst. Die Zähne wiesen ein sehr natürliches Aussehen auf (Fig. 1) und können ohne Verblendung direkt zur Herstellung von Prothesen eingesetzt werden.

**Tabelle 4: Farbe und Opazität zahnförmiger Formkörper**

| **Farbe*⁾** | **L*a*b*-Werte gemäß L*a*b*-Farbsystem** | | | **Opazität** |
|---|---|---|---|---|
| | **L** | **a** | **b** | |
| BL3 | 86.07 | 3.42 | 15.50 | 73.50 |
| A1 | 83.80 | 5.02 | 22.33 | 72.69 |
| A2 | 79.60 | 7.65 | 26.44 | 73.59 |
| A3 | 78.44 | 8.33 | 29.04 | 72.99 |
| A3, 5 | 76.65 | 9.53 | 31.40 | 73.56 |
| B1 | 82.51 | 3.83 | 21.43 | 73.88 |
| B3 | 78.03 | 7.41 | 32.00 | 73.41 |
| C2 | 75.62 | 6.26 | 25.43 | 75.55 |
| D2 | 77.79 | 5.89 | 22.31 | 74.04 |
| ^{*)} Farbbezeichnung gemäß Vita Farbschlüssel Lumin-VACCUM | | | | |

### Beispiel 3 (Vergleichsbeispiel)

### Vermessung herkömmlicher CAD-Discs

Die Farbe und die Opazität handelsüblicher PMMA Discs (PMMA-Telio CAD Disc, Fa. Wieland Dental) wurden auf die in Beispiel 1 beschriebene Weise gemessen. Die zur Messung benötigten Prüfkörper mit einem Durchmesser von 20 mm und einer Höhe von 2 ± 0,02 mm wurden aus den Rohlingen gefräst. Die Messwerte sind in Tabelle 5 angegeben. Die Discs weisen in allen Farbschattierungen eine deutlich höhere Opazität als erfindungsgemäße Rohlinge auf. Die Discs sind zur Herstellung von Kronen vorgesehen. Die Opazität ist so hoch, dass der Zahnstumpf genügend abgedeckt wird, nicht durchschimmert und sich nicht negativ auf die Farbe auswirkt.

Kunstzähne, die aus diesen Discs hergestellt werden, unterscheiden sich von Zähnen aus erfindungsgemäßen Materialien durch eine stumpfe Farbwirkung, die an das Aussehen von Knochen erinnert.

**Tabelle 5: Farbe und Opazität handelsüblicher PMMA-Discs¹⁾**

| **Farbe ²⁾** | **L*a*b*-Werte gemäß L*a*b*-Farbsystem** | | | **Opazität** |
|---|---|---|---|---|
| | **L** | **a** | **b** | |
| BL3 | 86.29 | 2.24 | 15.07 | 81.49 |
| A1 | 84.17 | 3.96 | 21.01 | 78.80 |
| A2 | 81.64 | 6.44 | 25.25 | 78.53 |
| A3 | 77.17 | 8.61 | 25.27 | 79.87 |
| A3,5 | 75.37 | 11.29 | 29.69 | 78.96 |
| B1 | 85.13 | 1.48 | 20.53 | 79.78 |
| B3 | 75.09 | 9.40 | 29.17 | 80.56 |
| C2 | 72.82 | 6.36 | 22.96 | 79.79 |
| D2 | 75.22 | 6.50 | 20.16 | 79.13 |
| ¹⁾ (PMMA-Telio CAD for Zenotec, Fa. Wieland Dental) | | | | |
| ²⁾ Farbbezeichnung gemäß Vita Farbschlüssel Lumin-VACCUM | | | | |

### Beispiel 4 (Vergleichsbeispiel)

### Vermessung herkömmlicher Kunststoffzähne

Auf die in Beispiel 1 beschriebene Weise wurden die Farbe und die Opazität der Schneide- und der Dentinschicht von konfektionierten Kunststoffzähnen (Zahnlinie SR Vivodent S DCL, Fa. Ivoclar Vivadent AG) gemessen. Die zur Messung benötigten Prüfkörper mit einem Durchmesser von 20 mm und einer Höhe von 2 ± 0,02 mm wurden aus den entsprechenden unpolymerisierten Massen hergestellt. Die Messwerte werden in den Tabellen 6 und 7 angegeben. Die Messwerte zeigen, dass die Dentinschicht eine sehr viel höhere und die Schneideschicht eine sehr viel geringere Opazität als die erfindungsgemäßen Materialien hat. Dennoch konnten mit den erfindungsgemäßen Materialen aus Beispiel 4 künstliche Zähne hergestellt werden, die in Ihrem Aussehen praktisch mit den handelsüblichen Zähnen übereinstimmen. In Figur 1 sind für 6 Farbschattierungen jeweils drei Zähne, die aus erfindungsgemäßen Rohlingen gefräst wurden, zusammen mit jeweils drei handelsüblichen Zähnen der gleichen Farbe abgebildet. Die Übereinstimmung ist verblüffend.

**Tabelle 6: Farbe und Opazität der Schneideschicht handelsüblicher Kunststoffzähne¹⁾**

| **Farbe ²⁾** | **L*a*b*-Werte gemäß L*a*b*-Farbsystem** | | | **Opazität** |
|---|---|---|---|---|
| | **L** | **a** | **b** | |
| BL3 | 89.99 | 2.03 | 12.58 | 55.71 |
| A1 | 90.12 | 1.13 | 14.47 | 52.29 |
| A2 | 89.44 | 1.94 | 16.90 | 54.20 |
| A3 | 89.35 | 1.35 | 19.87 | 52.97 |
| A3,5 | 87.97 | 2.57 | 25.37 | 53.90 |
| B1 | 90.00 | 0.46 | 14.87 | 53.55 |
| B3 | 89.40 | 0.75 | 20.36 | 53.04 |
| C2 | 87.40 | 1.55 | 21.68 | 53.73 |
| D2 | 87.35 | 2.87 | 17.62 | 54.17 |
| ¹⁾ Zahnlinie SR Vivodent S DCL, Fa. Ivoclar Vivadent AG | | | | |
| ²⁾ Farbbezeichnung gemäß Vita Farbschlüssel Lumin-VACCUM | | | | |

**Tabelle 7: Farbe und Opazität der Dentinschicht handelsüblicher Kunststoffzähne¹⁾**

| **Farbe ²⁾** | **L*a*b*-Werte gemäß L*a*b*-Farbsystem** | | | **Opazität** |
|---|---|---|---|---|
| | **L** | **a** | **b** | |
| BL3 | 85.50 | 2.88 | 13.18 | 85.88 |
| A1 | 82.24 | 4.25 | 20.45 | 83.21 |
| A2 | 78.55 | 6.85 | 26.76 | 84.63 |
| A3 | 75.81 | 7.29 | 27.34 | 84.62 |
| A3,5 | 73.57 | 9.98 | 31.59 | 84.73 |
| B1 | 82.36 | 2.34 | 20.05 | 84.12 |
| B3 | 74.81 | 8.37 | 33.71 | 83.48 |
| C2 | 70.82 | 6.02 | 24.89 | 85.80 |
| D2 | 74.28 | 5.14 | 20.20 | 85.64 |
| ¹⁾ Zahnlinie SR Vivodent S DCL, Fa. Ivoclar Vivadent AG | | | | |
| ²⁾ Farbbezeichnung gemäß Vita Farbschlüssel Lumin-VACCUM | | | | |

## Patentansprüche

1. Einfarbiger dentaler Formkörper, **dadurch gekennzeichnet, dass** er eine Opazität von 70 bis 78 % aufweist.

2. Formkörper nach Anspruch 1, der eine Opazität von 72 bis 76 % aufweist.

3. Formkörper nach einem der vorhergehenden Ansprüche, der eine polymerisierte Harzmatrix und Füllstoff enthält.

4. Formkörper nach Anspruch 3, bei dem die polymere Harzmatrix durch Polymerisation eines Monomers oder einer Mischung von Monomeren gebildet wird, das oder die aus
monofunktionellen Monomeren, vorzugsweise aus Methyl-, Ethyl-, 2-Hydroxyethyl-, Butyl-, Benzyl-, Tetrahydrofurfuryl-, Isobornyl-, p-Cumyl-phenoxyethylenglycol- (CMP-1E), Acetoxyacetylethyl-Methacrylat (AAEMA),
polyfuktionellen Monomeren, vorzugsweise aus Bisphenol-A-dimethacrylat, 2,2-Bis[4-(2-hydroxy-3-methacryloyloxypropyl)phenyl]propan (Bis-GMA; ein Additionsprodukt aus Methacrylsäure und Bisphenol-A-diglycidylether), ethoxy- oder propoxyliertem Bisphenol-A-Dimethacrylat, Bisphenol-A-Dimethacrylat SR-348c (Sartomer) mit 3 Ethoxygruppen, 2,2-Bis[4-(2-methacryloxypropoxy)phenyl]propan, 1,6-Bis-[2-methacryloyloxyethoxycarbonylamino]-2,2,4-trimethylhexan (UDMA; ein Additionsprodukt aus 2-Hydroxyethylmethacrylat und 2,2,4-Trimethylhexamethylendiisocyanat), TMX-UDMA (ein Additionsprodukt aus einer Mischung von HEMA und Hydroxypropylmethacrylat (HPMA) mit α,α,α',α'-Tetramethyl-m-xylylendiisocyanat (TMXDI)), Di-, Tri- oder Tetraethylenglycoldimethacrylat, Trimethylolpropantrimethacrylat, Pentaerythrittetramethacrylat, Glycerindi- oder -trimethacrylat, 1,4-Butandioldimethacrylat, 1,10-Decandioldimethacrylat (D₃MA), 1,12-Dodecandioldimethacrylat oder Bis(3-methacryloyloxymethyl)tricyclo-[5.2.1.0^{2,6}]decan (TCP), oder einer Mischung von mono- und polyfunktionellen Monomeren ausgewählt ist/sind.

5. Formkörper nach Anspruch 4, bei dem die polymere Harzmatrix durch Polymerisation einer Mischung von Methylmethacrylat, UDMA (oder TMX-UDMA) und (Mono)-Ethylenglycoldimethacrylat gebildet wird.

6. Formkörper nach einem der Ansprüche 3 bis 5, der als Füllstoff pulverförmiges ZrO₂ und/oder BaSO₄ enthält.

7. Formkörper nach einem der Ansprüche 3 bis 6, der als Füllstoff einen Füllstoff enthält, der eine geringere Opazität als die Harzmatrix aufweist.

8. Formkörper nach Anspruch 7, der als Füllstoff einen organischen Füllstoff, ein pulverförmiges Kompositmaterial (Kompositfüller) oder eine Mischung davon, vorzugsweise vernetzte Polymerpartikel enthält.

9. Formkörper nach einem der Ansprüche 3 bis 8, der ein Verdickungsmittel, vorzugsweise unvernetzte Polymerpartikel enthält.

10. Formkörper nach einem der Ansprüche 5 bis 9, der als Kompositfüller einen organisch-anorganischen Füllstoff enthält, der durch Härtung von Kompositpasten auf der Basis von Dimethacrylatmischungen und anorganischen Füllstoffen erhältlich ist, wobei das oder die Dimethacrylate aus Bis-GMA, (TMX-)UDMA und/oder D₃MA ausgewählt sind und die anorganischen Füllstoffe aus röntgenopaken Glasfüllstoffen und/oder Ytterbiumtrifluorid ausgewählt sind.

11. Formkörper nach einem der Ansprüche 3 bis 10, wobei das Verdickungsmittel eine mittlere Partikelgröße von 10 60 µm und der organische oder organisch-anorganische Füllstoff eine mittlere Teilchengröße von 2 bis 60 µm aufweist.

12. Formkörper nach einem der Ansprüche 3 bis 11 zur Verwendung als Rohling zur Herstellung von Dentalrestaurationen, der durch Härten der folgenden Mischung erhalten wird:
(1) 1 bis 20 Gew.-%, vorzugsweise 2 bis 10 Gew.-% und ganz besonders bevorzugt 3 bis 8 Gew.-% polyfunktionelle(s) radikalisch polymerisierbare(s) Monomer(e),
(2) 10 bis 60 Gew.-%, vorzugsweise 5 bis 50 Gew.-% und ganz besonders bevorzugt 10 bis 45 Gew.-% monofunktionelle(s) radikalisch polymerisierbare(s) Monomer(e),
(3) 0,01 bis 5,0 Gew.-%, vorzugsweise 0,1 bis 3,0 Gew.-% und ganz besonders bevorzugt 0,1 bis 2,0 Gew.-% Initiator für die radikalische Polymerisation,
(4) 20 bis 70 Gew.-%, vorzugsweise 30 bis 60 Gew.-% und ganz besonders bevorzugt 35 bis 55 Gew.-% Verdickungsmittel, vorzugsweise unvernetzte Polymerpartikel,
(5) 5 bis 50 Gew.-%, vorzugsweise 10 bis 40 Gew.-% und ganz besonders bevorzugt 15 bis 35 Gew.-% organischen Füllstoff, vorzugsweise vernetzte Polymerpartikel und ggf.
(6) 0,1 bis 5,0 Gew.-%, vorzugsweise 0,1 bis 2,0 Gew.-% und ganz besonders bevorzugt 0,2 bis 1,5 Gew.-% weitere Additiv(e), jeweils bezogen auf die Gesamtmasse der Mischung.

13. Formkörper nach einem der Ansprüche 1 bis 12, der eine der folgenden Farben aufweist (gemäß L*a*b*-Farbsystem):
| **Farbe** | **L*a*b* - Werte** | | |
|---|---|---|---|
| | **L** | **a** | **b** |
| **BL3** | 86.0 | 3.4 | 15.5 |
| **A1** | 83.8 | 5.0 | 22.3 |
| **A2** | 79.6 | 7.7 | 26.4 |
| **A3** | 78.5 | 8.3 | 29.0 |
| **A3,5** | 76.5 | 9.5 | 31.4 |
| **B1** | 82.5 | 3.8 | 21.4 |
| **B3** | 78.0 | 7.4 | 32.0 |
| **C2** | 75.6 | 6.3 | 25.4 |
| **D2** | 77.8 | 5.9 | 22.3 |

14. Verwendung eines Formkörpers gemäß einem der Ansprüche 1 bis 13 zur Herstellung von künstlichen Zähnen oder Zahnsegmenten.

15. Verwendung eines Formkörpers nach Anspruch 14, wobei die Herstellung der künstlichen Zähne oder der Zahnsegmente durch ein spanabhebendes Verfahren, vorzugsweise durch ein CAD/CAM-Verfahren erfolgt.

16. Verfahren zur Herstellung eines Formkörpers gemäß einem der Ansprüche 1 bis 13, bei dem man
(a) eine polymerisierbare Mischung bereitstellt, die radikalisch und/oder kationisch polymerisierbares Harz, ggf. Füllstoff und Initiator für die radikalische und/oder kationische Polymerisation enthält,
(b) man die Mischung durch die Zugabe von Farbmitteln einfärbt,
(c) man die Opazität der Mischung ggf. durch die Zugabe feinkörnigen Füllstoffs, vorzugsweise ZrO₂ und/oder BaSO₄ einstellt, und
(d) man die Mischung zu einem Formkörper formt und
(e) durch radikalische und/oder kationische Polymerisation härtet.

17. Verfahren nach Anspruch 16, bei dem man den Formkörper in Schritt (d) formt, indem man die Mischung durch Spritzgießen in eine Gießform einbringt und dann härtet.

18. Verfahren nach Anspruch 16, bei dem man den Formkörper in Schritt (d) durch ein generatives Verfahren formt und schichtweise härtet.
